# EUROPEAN PATENT APPLICATION

(11) **EP 2 138 508 A1**
(43) Date of publication of application: **30.12.2009**
(21) Application number: 08011562.9
(22) Date of filing: 26.06.2008
(51) Int. Cl.: C07K 14/195

(54) **Met agonists**

(71) Applicant: Helmholtz-Zentrum für Infektionsforschung GmbH, 38124 Braunschweig (DE)
(72) Inventor: Ferraris, Davide, 38102 Braunschweig (DE); Gherardi, Ermanno, Great Cambourne Cambridge CB23 5AT (GB); Heinz, Dirk, 38304 Wolfenbüttel (DE); Niemann, Hartmut, Jun. Prof., 33619 Bielefeld (DE)
(74) Representative: Kröncke, Rolf

(57) **Abstract**

The present invention relates to an isolated recombinant dimer derived from internalin B consisting of two polypeptides being covalently linked with each other. In particular, the present invention relates to an isolated recombinant dimer consisting of two polypeptides being covalently linked with each other whereby said polypeptides are derived from the internalin B (InIB) protein and the bonds are located in the LRR domain of said molecule and the monomeric polypeptides are arranged in an anti-parallel orientation. In a further aspect, the present invention relates to nucleic acid molecules encoding said peptides as well as pharmaceutical compositions comprising said peptides forming the dimer or the dimer itself. The dimer is particularly useful for wound healing or tissue regeneration after tissue damage.

## Description

The present invention relates to an isolated recombinant dimer derived from internalin B consisting of two polypeptides being covalently linked with each other. In particular, the present invention relates to an isolated recombinant dimer consisting of two polypeptides being covalently linked with each other whereby said polypeptides are derived from the internalin B (InIB) protein and the bonds are located in the LRR domain of said molecule and the monomeric polypeptides are arranged in an anti-parallel orientation. In a further aspect, the present invention relates to nucleic acid molecules encoding said peptides as well as pharmaceutical compositions comprising said peptides forming the dimer or the dimer itself. The dimer is particularly useful for wound healing or tissue regeneration after tissue damage.

### Background art

InIB is a surface protein 630 amino acid surface protein of the Gram-positive bacterium Listeria monocytogenes. It consists of an (i) N-terminal internalin domain comprising a Cap, a seven leucine rich repeats (LRRs) and the so called interrepeat (IR) region; (ii) a poorly characterized B-repeat; (iii) three C-terminal GW domains that can non-covalently anchor InIB on the surface of *Listeria* through interaction with lipoteichoic acid or interact with heparan sulfate proteoglycans (for an overview see H. Bierne and P. Cossart 2007, Microbes Infect. 9 (10) 1156-66). A substantial fraction of InIB is released from the bacterial surface. This form of InIB elicits a cellular response reminiscent of that caused by hepatocyte growth factor/scatter factor (HGF/SF) (K. Ireton, et al., 1999, J. Biol. Chem, 274 (24) 17025-32).

Due to the similar cellular response elicited by InIB compared to HGF-SF, the identification of the receptor tyrosine kinase Met as receptor for InIB was possible (Y. Shen, M. Naujokas, et al., 2000, Cell, 103 (3) 501-10). Normally, HGF/SF and Met mediate signals typical for cell survival and migration in embryongenesis and tissue regeneration but deregulation of Met also plays a major role in tumor invasion.

Two further receptors for InIB have been described, the complement receptor gC1qR (L. Braun, et al., 2000, EMBO J. 19 (7) 1458-66) and heparan sulphate proteoglycans (R. Jonquieres, et al., 2001, Mol. Microbiol, 42 (4) 955-65).

The crystal structures of the InIB have been characterized (M. Marino, et al.,1999, Mol. Cell 4 (6) 1063-72; W.D. Schubert, et al., 2001, J. Mol. Biol. 312 (4) 783-94;. M. Marino, M. et al., 2002 EMBO J 21 (21) 5623-24.). Characteristically, the LRR domain adopts a curved solenoid-like shape with an additional twist around the central superhelical axis. Recently, InIB in complex with its receptor, Met, has been published providing a detailed picture of the critical events during bacterial invasion (HH. Niemann, et al., 2007, Cell.130 (2) 235-46).

In the art it was shown that a fragment comprising Cap and LRR (InIB₂₄₁) is sufficient for Met binding (Shen *et al*., supra) and InIB₃₂₁, a fragment further comprising the IR region, is the minimal fragment capable of receptor activation (M. Banerjee, et al., 2004, Mol. Microbiol.52 (1) 257-71), namely phosphorylation of its receptor molecule. However, the InIB₃₂₁ fragment is not sufficient to induce a full biological response in target cells including cell division or cell motility (HH Niemann *et al.* supra)

The Met receptor itself was identified in the 1980s. Met is a two-chain, single-pass transmembrane helix receptor tyrosine kinase (RTK) and is part of a family of growth factor receptors. The extracellular portion of Met is composed of six distinct domains: an N-terminal Sema domain consisting of a 7-bladed β-propeller which shares sequence homology with domains found in Semaphorins and Plexins, a 50-residues cysteine-rich-PSI domain and 4 immunoglobuline like Ig domains (Ig1 - Ig4) linked to a single pass-α-helix spanning the membrane. Met is produced as a 1390 amino acid single-chain precursor that is cleaved by the cellular protease furin between residues 307 and 308 to yield a disulfide-linked two chain heterodimer. As noted before, the ligand of Met was identified as both a strong mitogen for hepatocytes (therefore the name hepatocyte growth factor or HGF), and a potent motility factor for epithelial cells (therefore the name scatter factor) (for an overview see C. Birchmeier, et al., 2003, Nat. Rev. Mol. Cell. Biol.4 (12) 915-25)

HGF/SF is a member of the plasminogen-related growth factor family and contains 728 residues. It is secreted as an inactive single chain precursor, which is proteolytically processed to form the biologically active disulfide linked α/β-heterodimer. The Sema domain of the Met receptor includes the binding site for the HGF/SF β-chain (J. Stamos et al., 2004, EMBO J. 23 (12) 2325-35). Two of the five domains within the HGF/SF α-chain are reported to bind to Sema as well (O. Holmes, et al., 2007, J. Mol. Biol.367 (2) 395-408), but have very recently also been suggested to bind the Met domains Ig3 and Ig4 (C. Basilico, et al., 2008, J. Biol. Chem. In press, available online with doi 10.1074/jbc.M800727200). Recently, Niemann et al., supra, reported the crystal structure of the InIB-Met complex. The concave surface of InIB is the main surface involved in Met recognition and binds to the Ig1 domain of Met through both hydrophobic and polar interactions. A second, less prominent interaction surface is constituted by the IR domain of InIB which interacts with the Sema domain of Met. Further, with binding experiments, the interaction between the InIB LRR and the Ig1 domain of Met was shown to contribute predominantly to the binding affinity while the contribution of the interaction between the IR domain of InIB and the Sema domain of Met is energetically negligible but necessary for proper activation of Met.

As mentioned before, the Met receptor is a member of the receptor tyrosine kinase family, representing a group of transmembrane glycoproteins that react to numerous extracellular signals and modulate an assortment of signalling pathways within cells, leading to cell proliferation, differentiation, migration or metabolic changes. Typically, the extracellular signal is transduced to the cytoplasm by autophosphorylating tyrosine residues on their catalytic cytoplasmic domain, thus, leading to the activation of Met and subsequent phosphorylation of downstream target involving signalling. Normally, said receptors are present as monomers in the cell membrane, however, after interaction with extracellular signals, formation of dimers or oligomers is observed, thus, leading to the activation of the receptor, in particular, initiating the autophosphorylation of the receptor (J. Schlessinger, 2000, Cell 03 (2) 211-25).

Said tyrosine autophosphorylation is crucial for recruitment and activation of a variety of signalling proteins. For the Met receptor various signalling pathways have been described eliciting diverse biological responses. In response to Met signals, characteristic cellular response is revoked, depending on the cell type in the culturing conditions. HGF and Met signals induce proliferative and anti-apoptotic responses in various cell types. A typical response is cell scattering, that is, having an increased motility and undergoing colony dispersal. Another typical phenomenon observed after Met signalling upon HGF stimulation are a so called branching morphogenesis, i.e. the formation of branched tubules.

In WO 2005/108424 the crystal structure of the complex of HGF/SF β-chain with Met receptor is described and the use of said complex for the design, identification and selection of ligands that modulate the Met receptor and in the interaction of HGF/SF with Met receptor is claimed therein. Further, the crystal structure of the Met receptor is described whereby the Met receptor is composed of the Sema domain and the PSI domain. The involvement of the Met receptor in various complex pathways in a cell including cell division, and breakdown of cell-cell contact and promotion of cell motility (jointly known as scattering), inhibition of apoptosis and tubule formation is known. Further, the Met receptor is essential during vertebrate development and contributes to organ development by promoting epithelial to mesenchymal transition (EMT). In EMT, cells leave their tissue and migrate through the body, a process known as invasive growth.

The main problem about HGF/SF is that the production of the recombinant protein is challenging and expensive as it requires expression in mammalian cell culture. Further, for activity HGF/SF needs to be converted from an inactive single chain to an active two-chain form by proteolytic cleavage by proteases in serum or tissues. Therefore, production in serum free-media is not possible. Fragments of HGF/SF produced in e.g. yeast showed lower biological activity than the full length HGF/SF.

Banerjee et al., supra describe mutants of InIB and their potency to activate Met. They found that full length InIB is able to promote Met phosphorylation at the same potency compared to HGF while the LRR domain alone is not able to induce Met phosphorylation even at a concentration of three orders of magnitude higher compared to InIB full length. Further studies showed that InIB is a stronger activator of certain pathways known for the Met receptor. Banerjee describes a purified, disulfide-linked dimeric LRR-only truncated form of InIB eliciting Met activation similarly to InIB full length, while the monomer did not show Met activation. Said disulphide linked dimeric form of InIB was connected with a single disulfide bond formed by the only cysteine present next to the LRR domain, namely, Cys242. It is noted therein that the LRR fragment does not exist physiologically and its dimerisation through a disulfide on a Cys242 is found to be an artefact of truncation and purification in the absence of reducing agent.

Antagonists and agonists of the Met receptor are thought to be particularly useful for the treatment or prevention of various diseases. For example, inhibitors of the Met signalling may block EMT like processes of cancer metastasis and contribute to effective cancer therapy. In addition, scattering of cells may be inhibited.

In contrast, agonists may be useful in regeneration processes of tissue damages, like wounds, damages of heart tissue, liver tissue, skin, kidney tissue, lung tissue and other tissue injuries, etc. Further, said agonist may be helpful for stimulating cell division in vitro and in order to stimulate tissue regeneration, e.g. after transplantation.

Thus, a first object of the present invention is to provide new agonists of the Met receptor beneficial in tissue regeneration. In particular, the object of the present invention is to provide an agonist or activator of the Met mediated signalling pathway(s) whose production is economical and feasible allowing its commercial use, in particular as a pharmaceutical having sufficient shelf-live.

Another problem to be solved by the present invention is the provision of pharmaceutical composition containing said agonists. A further problem to be solved by the present invention is providing methods for the treatment or prevention of tissue damages or for tissue regeneration.

### Brief description of the invention

The present invention relates to isolated recombinant internalin B (InIB) dimer consisting of two monomeric polypeptides being covalently linked with each other via at least two bonds selected from disulfide bonds, direct bonds or other covalent linkages whereby said polypeptides are derived from the InIB protein and the bonds are located in the LRR domain corresponding to polypeptides of said protein of amino acids 81 to 241 of InIB of Seq. ID No. 1 wherein the monomeric polypeptides are arranged in an anti-parallel orientation.

In a preferred embodiment, said dimer is an agonist of the Met receptor activating the Met receptor mediated signalling pathways in cells.

Preferably, said dimer contains at least two cysteine residues present in the LRR region of each monomeric polypeptide whereby said two cysteines are introduced by genetic engineering. Each of the two cysteines forms a disulfide bond with a cysteine present in the other polypeptide, thus, forming the dimer of the present invention having anti-parallel orientation. Particularly preferred are dimers of monomeric polypeptides according to Seq. ID Nos. 2 to 4. Said dimers further comprise aromatic amino acid residues at positions 104, 124, 170, 214 of Seq. ID No. 1 which assist in interaction with the Met receptor.

In a further aspect, the present invention relates to a nucleic acid molecule encoding a monomeric polypeptide according to the present invention, in particular Seq. ID No.7.

Another aspect of the present invention concerns pharmaceutical compositions comprising a dimer according to the present invention. Said dimer is particularly useful for wound healing or tissue regeneration after tissue damage. In particular, said dimer is useful for the treatment of tissue damages, in particular, skin or mucosal tissue damages but also damages or injuries in liver, lung or heart muscle. Said tissue injuries or damages may occur in the cause of progressing diabetes.

Finally, said dimer is useful as a proliferating agent stimulating or enhancing cell proliferation in particular, for tissue engineering techniques.

### Brief description of the drawings

Figure 1 shows structures and domains of InIB₂₄₁ (top) and InIB₃₂₁ (bottom) and their representations used herein (right).
Figure 2 provides a model of a dimer according to the present invention. Figure 2a is a crystal structure of the InIB₃₂₁ "crystal dimer". Figure 2b shows the dimer interface of the InIB₃₂₁ "crystal dimer". G206C/A227C mutations are represented in sticks. Distances between main chains are 3.8 A.
Figure 3 provides the results of the scattering tests. In figure 3a the data for the assay performed on MDCK cells are shown. The reported concentrations are the minimum concentration at which cell scattering was clearly visible. The highest ligand concentration used in the experiment was 1x10⁻⁵ M. Figure 3b is a bar graph of scattering assay performed on MDCK cells. The higher the bar, the higher the scattering potency.
Figure 4 shows the model of the InIB₃₂₁ dimer according to the present invention. Figure 4a shows the positions of stabilising interactions in the InIB-InIB dimer interface. Figure 4b: Atomic view of the InIB-InIB interface stabilising interactions as viewed in the InB_Crys dimer structure Left: interactions between Arg 184, Asp 263 and Ser 183 in InIB₃₂₁_Crys structure. Right: Salt bridges between Asp 200 and Arg 224 in InIB "crystal dimer" structure. In these two pictures, the intermolecular disulfide bond is visible in the background.
Figure 5 demonstrates the effect of the dimer according to present invention on the induction of cell proliferation in comparison to the full length InIB molecule and the monomeric InIB₃₂₁ peptide. For reference, HGS/SF is used.

### Detailed description of the present invention

The present invention relates in a first aspect to an isolated, recombinant internalin B (InIB) dimer consisting of two monomeric polypeptides being covalently linked with each other via at least two bonds selected from disulfide bonds, direct bonds or other covalent linkages whereby said polypeptides are derived from the lnIB protein and the bonds are located in the LRR domain corresponding to polypeptides of said protein of amino acids 81 to 241 of InIB of Seq. ID No. 1 wherein the monomeric polypeptides are arranged in an anti-parallel orientation.

Preferably, said dimer has at least two cysteines present in the LRR domain of each monomeric polypeptide whereby said two cysteines are preferably introduced by genetic engineering. Said cysteines form two disulfide bonds with the two cysteines present in the second or other polypeptide forming the dimer.

Of course, more than two covalent bonds may exist between the polypeptides forming the dimer, like 3, 4, or 5 bonds.

Said dimer may act as an agonist of the Met receptor, in particular of the Met receptor in mammalian species, like humans.

### In this connection, the following definitions apply herein:

In the following, "AA" or "aa" is used as abbreviations for amino acids; amino acids are represented by single letter code or three letter code.

The term "InIB derivative" as used herein, refers to a polypeptide that has a different sequence than the reference InIB polypeptide. In some embodiments, a derivative has at least 80 % amino acid identity with the InIB derivatives of amino acid sequence shown in Seq. ID No. 3 or 4. The derivatives include those polypeptides that have substitutions, additions, or deletions. The derivative also includes those polypeptides that have at least one conservative amino acid substitution, preferably, all of the substitutions are conservative. Preferably, said derivatives do not contain any other cysteine residue than those necessary for forming the bonds between the two polypeptides of the dimer according to the present invention.

In some embodiments, the InIB derivative has about 1-25 conservative amino acid substitutions, more preferably about 1-20 conservative amino acids substitutions, more preferably about 1-10 conservative amino acid substitutions, more preferably about 1-5 conservative amino acid substitutions, and more preferably about 1-2 conservative amino acid substitutions. The derivatives have the biological activity of binding to the Met receptor and activating it in a manner similar to the activity of the dimer formed by two polypeptides of Seq. ID No. 4. Ordinarily a polypeptide derivative derived form InIB will have at least 80% sequence identity; like at least 90% sequence identity, like at least 94% sequence identity, like at least 95% sequence identity, like at least 98% sequence identity, like at least 99% sequence identity with a InIB polypeptide having an amino acid sequence comprising Seq-ID No. 3 or Seq-ID No. 4.

The term "corresponding" or "corresponds" refers to an amino acid residue or amino acid sequence that is found at the same positions or positions in a sequence aligned with a reference sequence. It will be appreciated that when the amino acid position or sequence is aligned with the reference sequence the numbering of the amino acids may differ from that of the reference sequence or a different numbering system may be utilized.

"Structural homolog" of the dimer of the present invention as used herein refers to a dimer containing polypeptides covalently linked with each other that contains one or more amino acid substitutions, deletions, additions, or rearrangements with respect to the amino acid sequence of the polypeptides of Seq. ID. No. 3 or 4, but that, when folded into its native conformation and after forming the dimer, exhibits or is reasonably expected to exhibit the tertiary (three-dimensional) structure of the dimer consisting of two polypeptides of Seq ID No. 4. For example, structurally homologous molecules of the dimer according to the present invention include InIB derivatives, preferably derivatives with one or more conservative amino acid substitutions as defined above. In some embodiments, the InIB derivative has only conservative amino acid substitutions. Homology of the tertiary structure can be probed, measured, or confirmed by known analytic or diagnostic methods, for example, X-ray crystallography, NMR, circular dichroism, a panel of monoclonal antibodies that recognize the dimer, and like techniques. For example, structurally homologous molecules can have substitutions, deletions or additions of one or more contiguous amino acids, such as loop or a domain. Structurally homologous molecules also include "modified" dimer molecules that have been chemically or enzymatically derivatized at one or more constituent amino acid, including side chain modifications, backbone modifications, and N- and C-terminal modifications including acetylation, hydroxylation, methylation, amidation, and the attachment of carbohydrate or lipid moieties, cofactors, and like modifications.

The present disclosure also includes said derivatives of the dimer and the polypeptides forming said dimer as defined above. In some embodiments, said derivatives has at least 70%, like 80% such as 90% sequence identity to SEQ ID NO: 3 or 4, and has changes at amino acids residues other than those specified below, in particular, the aromatic amino acid residues 104, 120, 170, and 214 of SEQ ID No. 1, preferably the amino acid changes are only conservative substitutions.

Amino acid substitutions include one or more conservative amino acid substitutions. The term "conservative" amino acid substitution as used herein refers to an amino acid substitutions which substitutes a functionally equivalent amino acid. Conservative amino acid changes typically result in silent changes in secondary structure of the amino acid sequence of the resulting polypeptide. For example, one or more amino acids of a similar polarity act as functional equivalents and results in a silent alteration within the amino acid sequence of the peptide. In general, substitutions within a group can be considered conservative with respect to structure and function. However, the skilled artisan will recognize that the role of a particular residue is determined by its context within the three-dimensional structure of the molecule in which it occurs. The long aliphatic portion of the Arg side chain can constitute a feature of its structural of functional role, and this may be best conserved by substitution of a nonpolar, rather than another basic residue. Also, it will be recognized that side chains containing aromatic groups (Trp, Tyr, and Phe) can participate in ionic-aromatic or "cation-pi" interactions. In these cases, substitution of one of these side chains with a member of the acidic or uncharged polar group may be conservative with respect to structure and function.

Amino acid substitutions can be the result of replacing one amino acid with another amino acid having similar structural and/or chemical properties such as the replacement of a leucine with an isoleucine, i.e., conservative amino acid replacement. Examples of conservative substitutions are shown in the Table below.

The variation allowed can be determined by systematically making insertions, deletions or substitutions of amino acids in the sequence and testing the resulting variants for activity exhibited by the native sequence.

| **Original Residue** | **Exemplary Substitutions** | **Preferred Substitutions** |
|---|---|---|
| Ala (A) | Val; Leu; Ile | Val |
| Arg ( R ) | Lys; Gln; Asn | Lys |
| Asn (N) | Gln; His; Asp; Lys; Arg | Gln |
| Asp (D) | Glu; Asn | Glu |
| Cys ( C ) | Ser; Ala | Ser |
| Gln (Q) | Asn; Glu | Asn |
| Glu ( E ) | Asp; Gln | Asp |
| Gly (G) | Ala | Ala |
| His (H) | Asn; Gln; Lys; Arg Leu; Val; Met; Ala; Phe; | Arg |
| Ile (I) | Norleucine Norleucine, Ile; Val; Met; | Leu |
| Leu (L) | Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser Ile; Leu; Met; Phe; Ala; | Phe |
| Val (V) | Norleucine | Leu |

As used herein, the term "agonist" or "activator" refers to compounds that are able to activate the signalling cascade, and, thus, the signalling pathway involving the Met receptor, said activation is defined in the ability to induce DNA-synthesis and/or scattering of cells expressing the Met receptors. Said cells being activated similar to the activation of the cells in the presence of sufficient amounts of HGF/SF.

As used herein, the term "LRR domain" or "LRR region" refers to amino acid residues 81 to 241 of Seq. ID No. 1.

The term "homology" as used herein means a value obtained by a BLAST (Basic local alignment search tool; Altschul, S. F. et al., J. Mol. Biol., 215, 403-410, (1990)) search. The homology in the amino acid sequence may be calculated by a BLAST search algorithm. More particularly, it may be calculated using b12seq program (Tatjana A. Tatusova and Thomas L. Madden, FEMS Microbiol. Lett., 174, 247-250, 1999) in a BLAST package (sgi32bit edition, version 2.0.12; obtained from NCBI) in accordance with a default parameter. As a pairwise alignment parameter, a program "blastp" is used. Further, "0" as a Gap elongation cost value, "SEG" as a filter for a Query sequence, and "BLOSUM62" as a Matrix are used, respectively.

Preferably, the dimer according to the present invention is a dimer wherein any naturally occurring cysteine residues present in the InIB protein of Seq. ID No. 1 have been mutated to any other amino acids, preferably, to the amino acid alanine. In particular, it is preferred that the cysteine residue at position 242 is mutated to alanine. Most preferably, all naturally occurring cysteins are mutated to amino acid residues not forming disulfide bonds or other covalent bonds when forming the dimer.

Of course, it is possible that the monomeric polypeptides are not identical but different to each other. However, it is important that said monomeric polypeptides are arranged in an anti-parallel orientation.

Particular preferred, the monomeric polypeptide comprises aromatic amino acid residues at positions 104, 124, 170 and 214 of Seq. ID No. 1, preferably F104, W124, Y170 and Y214.

Preferably, the mutation of aa residues to cysteine residues are with the aa residues present on the convex side of the LRR region of the InIB derivative. Further preferred, the cysteines introduced in the molecule for the building of disulfide bonds are present in two adjacent helices of the LRR domain of the InIB derivative, in particular, in helices 5 and 6, respectively.

The cysteine residues introduced into the monomeric polypeptides forming the dimer of the present invention are in particular G206C and A 227C.

In a most preferred embodiment, the monomeric polypeptide is the polypeptide of Seq. ID No. 3 or Seq. ID No. 4, namely, the InIB₃₂₁ fragment having the following mutations: G206C, A227C and C242A. In the following, the dimer formed from the mutated InIB₃₂₁ molecule having C242A, G206C and A227C is also referred to as "crystal dimer".

In another embodiment, the dimer according to the present invention is an agonist or activator of the Met receptor resulting in the activation of the Met receptor mediated signalling pathway including scattering of the cells and/or DNA synthesis.

Particularly preferred, the dimer according to the present invention is formed from two monomeric polypeptides which polypeptides are a peptide of Seq. ID No. 3 or 4; a polypeptide comprising an amino acid sequence of Seq. ID No. 3 or 4 and exhibiting a binding to the Ig1 domain of the Met receptor; a functional fragment or derivative of said polypeptide exhibiting binding to the Ig1 domain of the Met receptor or a polypeptide having a 70 % or more homology with an amino acid sequence of the Seq. ID No. 3 or 4 or a functional fragment or derivative thereof whereby the cysteine residues in Seq. ID No. 3 or 4 and the aromatic amino acid residues at positions 104, 124, 170, and 214 remain unchanged; or an altered polypeptide in which at least parts of the amino acids have been replaced by analogous parts of a different specificity, in particular, human, while maintaining the ability to bind Ig1 domain of the Met receptor and to form dimers in an anti-parallel confirmation wherein the cysteine residues of Seq. ID Nos. 3 or 4 and the aromatic amino acid residues at position 104, 124 170, and 214 of Seq. ID Nos. 3 and 4 remain unchanged.

Depending on the species, the monomeric polypeptide may be modified in that at least parts of the amino acids are replaced by analogous parts of a different specificity, in particular, by analogous parts of human, while maintaining the agonistic activity. That is, the dimer may be provided in a "humanized" form. A humanized form means that the ability of the dimer to elicit an immune response in the species administered to is reduced or diminished. To minimize the risk of eliciting an unwanted immune response in a subject to be treated, it is known in the art to adapt said molecule in particular to adapt polypeptide to the species administered to. For example, for monoclonal antibodies humanisation is well established. The same is true for other compounds stemming from polypeptides of other species. The skilled person is well aware of techniques and methods for adopting the dimer according to the present invention to other species, thus, diminishing an immune response, and to provide humanised dimers.

In another aspect, the present invention relates to a nucleic acid molecule encoding the monomeric polypeptide of the dimer according to the present invention. Depending on the species, the nucleic acid sequences of encoding the monomeric polypeptide of the dimers according to the present invention are composed following the genetic code of said species as known in the art. In a preferred embodiment, the nucleic acid is the molecule of Seq. ID No. 7.

In addition, the present invention relates to pharmaceutical compositions comprising the dimer according to the present invention. Optionally, diluents, carriers or other pharmaceutical acceptable molecules known to be present in typical pharmaceutical compositions are present.

Said pharmaceutical composition is particularly useful for wound healing or tissue regeneration after tissue damage or injuries.

The process of wound healing or tissue regeneration after tissue damage requires the activity of growth factors and other cytokines. Met is the receptor for one of these growth factors, namely the HGF/SF molecule. As mentioned before, HGF/SF is an important mitogen for liver cells and induces cell motility. Further, HGF/SF induced Met signalling is required for the regeneration of several tissues. For example, it is described in the art that the physiological repair of skin wounds after injury requires HGF/SF and Met. In addition, it is described in the art, that HGF/SF promotes other components of the repair process, e.g. re-epithelialisation, angiogenesis and connective tissue formation. Further, a function of the HGF/SF and Met pathway is described for the regeneration of kidney and lung and other tissues.

The main problem about HGF/SF is that the production of the recombinant protein is challenging and expensive as it requires expression in mammalian cell culture. Further, for activity HGF/SF needs to be converted from an inactive single chain to an active two-chain form by proteolytic cleavage by proteases in serum or tissues. Therefore, production in serum free-media is not possible. Fragments of HGF/SF produced in e.g. yeast showed lower biological activity than the full length HGF/SF. In contrast, the dimer according to the present invention can be produced easily with high yields and high amounts in well established production systems, e.g. E. coli or yeast, or mammalian cell culture systems.

Thus, molecules able to substitute HGF/SF and allowing Met signalling are beneficial for the treatment of respective diseases.

That is, the dimers according to the present invention which are easy to produce and stable allowing long storage of the same are beneficial in order to promote tissue repair, in particular, liver repair after acute or chronic liver injury. Further, said agonists are useful for treatment of tissue damages, like wounds, in particular in the group of patients with chronic and poorly healing skin wounds. These patients occurs for example in the group of people suffering from diabetes which now receive palliative treatment which is often insufficient to prevent complications of the wound and not infrequently requires amputation of the areas affected therewith.

With respect to skin and other mucosal tissues, the dimer according to the present invention is useful for the regeneration of said mucosal tissue, in particular, of skin. For example, said molecules are helpful for treating chronic-lung disease (for example the so called chronic obstructive pulmonary disease or COPD).

If necessary, the compounds are formulated or modified in a way allowing for example delivery to small bronchi via the airways when treating lung damages. Alternatively, when applying to skin, a topical preparation may be provided. Another possibility for the use of a pharmaceutical composition comprising the dimer according to the present invention is the repair of damaged heart tissue, in particular heart muscle tissue. This may be in particular useful for post-infarct heart repair.

The composition comprising the agonist will be formulated, dosed, and administered in a fashion consistent with good medical practice. Factors for consideration in this context include the stage of the particular disease or disorder being treated, the particular mammal being treated, the clinical condition of the individual subject, the site of delivery of the agent, the method of administration, the scheduling of administration, and other factors known to medical practitioners. The effective amount of the agonist to be administered will be governed by such considerations. The key factor in selecting an appropriate dose and scheduling is the result obtained, as indicated above. The agonist is administered by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated.

"Therapeutically- or pharmaceutically-effective amount" as applied to the compositions of the instant invention refers to the amount of composition sufficient to induce a desired biological result. That result can be alleviation of the signs, symptoms, or causes of a disease, or any other desired alteration of a biological system. In the present invention, the result will typically involve an increase in the DNA-synthesis of cells expressing the Met receptor.

In vitro assays may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" means administration of a therapeutically effective dose of the aforementioned pharmaceutical composition comprising the dimer according to the present invention in form of salts and solvates thereof to an individual.

The pharmaceutical composition according to the present invention comprises the dimer as described herein and, optionally, a pharmaceutical acceptable carrier. Such pharmaceutical compositions comprise a therapeutically effective amount of the dimer and, optionally, a pharmaceutically acceptable carrier. The pharmaceutical composition may be administered with a physiologically acceptable carrier to a patient, as described herein. Acceptable means that the carrier be acceptable in the sense of being compatible with the other ingredients of the composition and not be deleterious to the recipient thereof. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, aerosols and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds, salts or solvates thereof, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in a unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The attending physician and clinician will determine the dosage regimen. A typical dose can be, for example, in the range of 0.001 to 1000 µg; however, doses below or above this exemplary range are envisioned, especially considering the aforementioned factors.

These and other embodiments are disclosed and encompassed by the description and examples of the present invention. Further literature concerning any one of the methods, uses and compounds to be employed in accordance with the present invention may be retrieved from public libraries, using for example electronic devices.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington 's Pharmaceutical Sciences 16th edition, Osol, A. Ed. (1980). Sustained-release preparations may be prepared. Suitable examples of sustained- release preparations include semipermeable matrices of solid hydrophobic polymers containing the antagonist, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene- vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydiOxybutyric acid. The formulations to be used for in vivo administration must be sterile. This is readily accomplished by filtration through sterile filtration membranes.

Preferably, the dimer according to the present invention is provided in form of topical formulations, aerosols or other formulations suitable for intake via the bronchi, or in form of injections suitable for direct application to the tissue to be treated, e.g. the heart muscle, liver, kidney, etc. For example, the dimer may be present as active ingredient in a plaster when used for wound healing. Of course other forms are suitable, like cream, salve, ointment, tonic, etc.

Finally, the dimer according to the present invention is useful as a proliferation inducing factor in cell culture. In particular, said dimer is applicable for tissue engineering in vitro or in vivo. For example, the dimer can substitute HGF/SF in the generation of artificial liver tissue or skin. In particular, after heavy burns, the patient receives skin transplants which have been generated in vitro.

The following examples are intended to illustrate the invention further, however, they are not meant to limit the scope of the invention in a new way.

### Examples

### Example 1 Design and preparation of artificial dimers of InIB

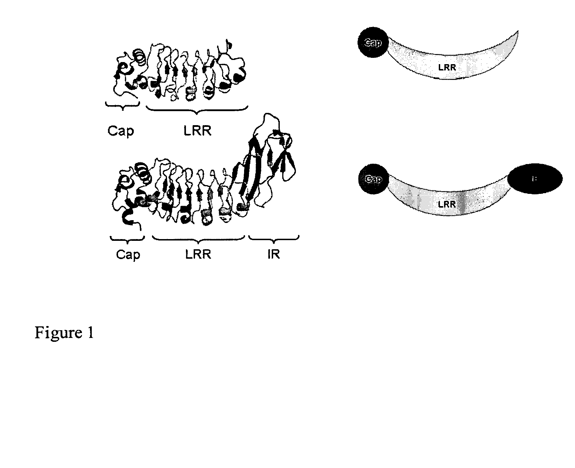

The design of InIB disulfide-linked dimers through mutagenesis requires a careful selection of the residues to mutate. For the selection of the best candidates, the following criteria have been applied: 1) mutated residues must be on the surface of the protein and not buried, 2) they must lie outside the binding interfaces shared by InIB and Met, 3) predicted disulfide bond(s) must be "likely" and not excessively stretched. Mutations were performed on InIB₃₂₁ (residues 36-321 of Seq. ID No. 1 having the Cap, LRR and IR-domain, known to bind and partially activate Met) and InIB₂₄₁, see figure 1.

A crystal contact has been identified that suited the needs occurring between the convex sides of LRR domains of a pair of InIB molecules symmetry related by a twofold axis. In this arrangement, the cognate InIB molecules lie approximately on the same plane. Initially, this contact has been identified in two crystal forms of InIB₃₂₁, a triclinic and a rhombohedral one. Only later this contact has been found in further crystal forms, e.g. the crystal form II of InIB₃₂₁ in complex with Met (Niemann *et al.* supra; coordinates deposited in the Protein Data Bank under accession code 2uzy). The recurrence of this crystal contact in different crystal forms of InIB suggests it to be a favourable, low-energy arrangement.

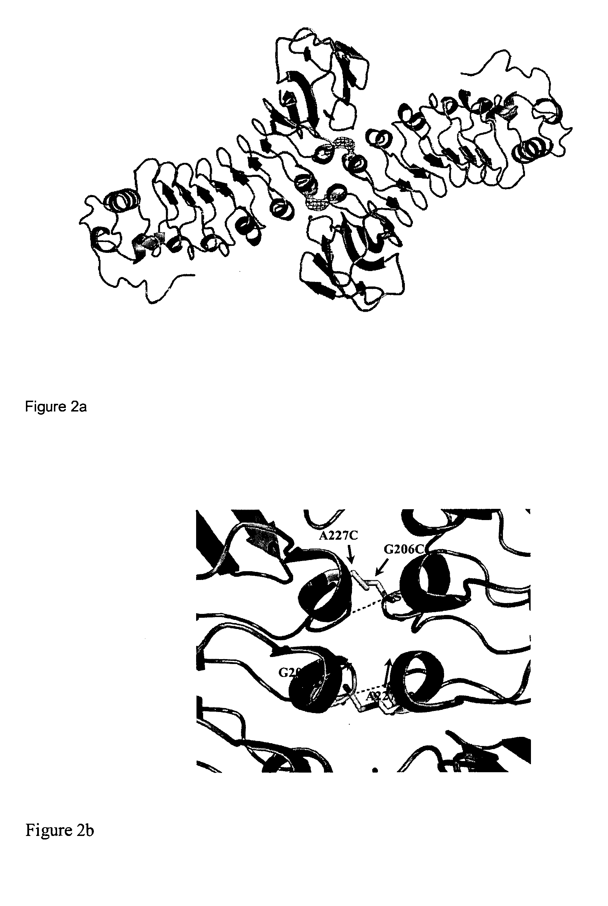

For recreating this crystal contact it has been searched for two amino acids located in the dimer interface that were sufficiently close to the opposite, symmetry-related chains so that, upon mutation of the two residues into Cys, two disulfide bonds could easily be formed. Inspection of the dimer interface showed helices 5 and 6 which were, in the shortest distance, 3.2 A apart from the opposite chain, and therefore compatible with the length of a disulfide bond. The closest residues to this region were Gly 206 and Ala 227. It has been predicted that mutation of these residues into Cys would easily promote the formation of intermolecular disulfide bonds with their spatially opposite residues (Cys 206 of chain A with Cys 227 of chain B and *vice versa*). Distances between the mutated residues and their allowed rotamers suggested the likeliness of intermolecular disulfide bonds formation, see figure 2.

In addition, mutation of the naturally occurring Cys at position 242 into an Ala was performed. The C242A mutation was carried out with the intention of excluding the formation of undesired intramolecular disulfide bonds other than the desired ones. Banerjee *et al.* supra showed that this mutation does not impair Met binding.

Various disulfide linked dimers of InIB₂₄₁ (Cap and LRR domain) and InIB₃₂₁ (Cap domain + LRR + IR domain) have been designed with the intent to investigate the role of dimerization and the relative spatial arrangement of the Met receptor during activation. Moreover, the role of the rigidity of the whole InIB-Met complex upon activation was investigated through the engineering of a double disulfide-linked rigid InIB₃₂₁ dimer. Table 1 shows the list of the engineered dimers, the mutations performed and their graphical representation used throughout the text.

**Table 1 Engineered disulfide cross-linked InIB artificial dimers used herein**

| InIB mutants | Dimer type | Representation |
|---|---|---|
| InIB241 K64C | Head-to-Head | 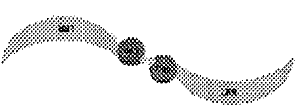 |
| InIB241 N158C | Back-to-Back | 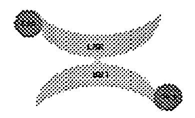 |
| InIB242 | Tail-to-Tail | 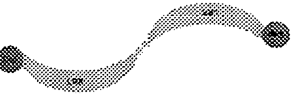 |
| InIB321 K64C | Head-to-Head | 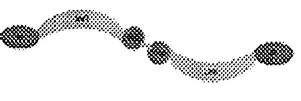 |
| InIB321 N158C | Back-to-Back | 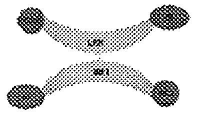 |
| InIB321 K280C | Tail-to-Tail | 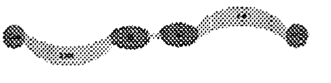 |
| InIB321 G206C A227C | "Crystal dimer" | 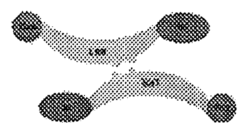 |

### Purification of InIB dimers

All InIB constructs and their mutants were expressed in *E. coli* BL21 + cells grown in LB medium and induced at 20° for about 16 hours. Following cell disruption by French Press, the lysate was incubated with suitable amount of GS Sepharose resin and washed extensively prior to digestion with TEV protease. The eluted proteins showed generally a good purity already after affinity chromatography. Cysteines were oxidized through dialysis of the protein solution against a buffer containing 1 mM H₂O₂ as the oxidizing agent.

Further purification was performed using an anion exchange column. Monomeric and dimeric species eluted separately from the column following a linear salt gradient.

After ion exchange chromatography, fractions corresponding to the dimeric species were pooled and purified through a gel filtration column.
A second, faster protocol was also applied for the purification of the protein dimers. Following affinity chromatography and dialysis, the mixture of monomeric and dimeric species were filtered and eluted through a gel filtration column. The different retention volumes of the monomeric and dimeric species allowed their complete separation.

### Purification of InIB₃₂₁ "crystal dimer"

Since the presence of two cysteines could prime the formation of undesired InIB multimers, special attention was dedicated to the purification of the InIB_{321_}Crys dimer, and particular care was put in the assessment of the oligomerization state of the purified species.

As for the other mutants, purification of InIB₃₂₁ "crystal dimer" using ion exchange chromatography caused the separation of monomeric and dimeric forms. No peaks of multimeric species were detected, thus ruling out the presence of higher oligomers. Fractions belonging to the dimeric species were pooled and eluted through a gel filtration column and the relative molecular mass was calculated according to the elution of globular protein standards. The presence of one single peak at the expected molecular weight ruled out the presence of higher oligomers. MALDI-TOF analysis showed two peaks, representing the singly and doubly charged species of an InIB₃₂₁ dimer, thus excluding the presence of higher aggregates. The molecular mass of the singly-charged species was in agreement with the predicted one (32122.8 Da).

### Summary of purification of InIB dimers

Purity and oligomerization state of all the dimers were finally assessed using reducing and non-reducing SDS-PAGE gels. Non-reducing gels clearly showed the presence of InIB dimers with no presence of higher oligomers. The different migration in the gel of the single proteins might be due to the different migration properties of the dimers through the acrylamide mesh. Addition of DTT to the sample buffer caused complete reduction of the cysteines, thus forming monomeric species only and proving the nature of the intermolecular disulfide bond.

The relative molecular masses of monomers and dimers of InIB were estimated through analytical gel filtration. All proteins eluted through the column at the expected molecular mass.

Table 2 summarizes the results of InIB₂₄₁ and InIB₃₂, dimers purification together with the mutations performed, buffer used for storage, the solubility and the oligomerization properties as assessed by SDS-PAGE gel and gel filtration chromatography.

**Table 2 Summary of purified InIB₂₄, and InIB₃₂₁ and dimers.**

| **Name** | **Mutation(s)** | **Domains** | **Type of dimer** | **Buffer** | **Solubility** | **SDS-PAGE** | **Gel filtration** |
|---|---|---|---|---|---|---|---|
| InIB241_HH | K64C | Cap+LRR | Head-to-Head | 10 mM Tris pH=8 | MaK solubility | Dimer | Dimer |
| InIB241_BB | N158 | Cap+LRR | Back-to-Back | 10 mM Tris pH=B 300 mM NaCl | Max soluble ∼4.5 mg/ml | Dimer | Dimer |
| InIB242_TT | (truncated) | Cep+LRR | Tail-to-Tail | 10 mM Tris pH=8 300 mM NaCl | soluble | Dimer | Dimer |
| InIB321_HB | K64C C242A | Cap+LRR+IR | Head-to-Head | 10 mM Tris pH=8 300 mM NaCl | Max solubility ∼2 mg/ml | Dimer | Dimer |
| InIB321_BB | M156C C242A | Cap+LRR+IR | Back-to-Back | 10 mM Tris pH=8 150 mM NaCl | soluble | Dimer | Dimer |
| InIB321_TT | K280C C242A | Cap+LRR+IR | Tail-to-Tail | 10 mM Tris pH=8 150 mM NaCl | soluble | Dimer | Dimer |
| InIB321_Crys | G206C A227C C242A | Cap+LRR+IR | "Crystal dimer" | 10 mM Tris pH=8 150 mM NaCl | soluble | Dimer | Dimer |

### Example 2 Activity of InIB artificial dimers towards Met activation

### Cell scattering assay

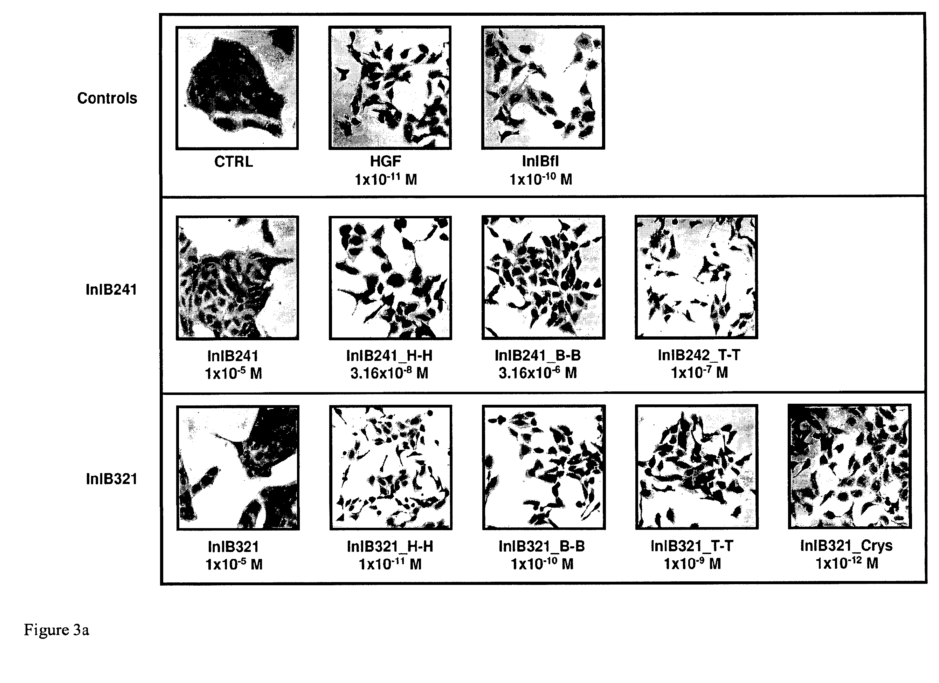

Next, the activity of the InIB₂₄₁ and InIB₃₂₁ dimers towards Met activation has been tested. One standard evaluation for Met activity is the cell scattering assay performed using MDCK cells following overnight incubation with the desired ligand(s) (M. Stoker, et al., 1987, Nature 327 (6119) 239-42). The capability of the present InIB artificial dimers to promote cell scattering was compared to that of the physiological Met ligand HGF and that of InIB full length (InlBfl). Figure 3a shows representative images of cells scattering obtained for different ligand types and concentrations. That is, the Scattering assay was performed as described by Stoker et al., 1987, supra.

The experiment was performed using a treated 96 well-plate Nunc Nunclon 96 MicroWell™ Plates. MDCK cells were routinely cultured in DMEM 5% FCS + 1% P&S. Subconfluent cells were diluted to a density of 3.3 x 10⁴ cells/ml and 150 µl were aliquoted in each well with a final cell density of 5 x 103 cells/well and incubated overnight for allowing their adherence to the bottom of the plate. The following day, proteins were diluted in DMEM 5% FCS + 1% P&S at the suitable concentrations following a serial semilog dilution and added to the cells following overnight incubation at 37°C and 5% CO2. The following day the cells were carefully and repeatedly washed with PBS and fixed with 10% formaldheyde in PBS for 5 minutes and then stained with a solution containing 2 g/l of Coomassie Blue R-250 in water/methanol/acetic acid, 50/40/10. Stained cells were again washed repeatedly and examined on an inverted microscope (Axiovert 153TV, Zeiss), and results scored.

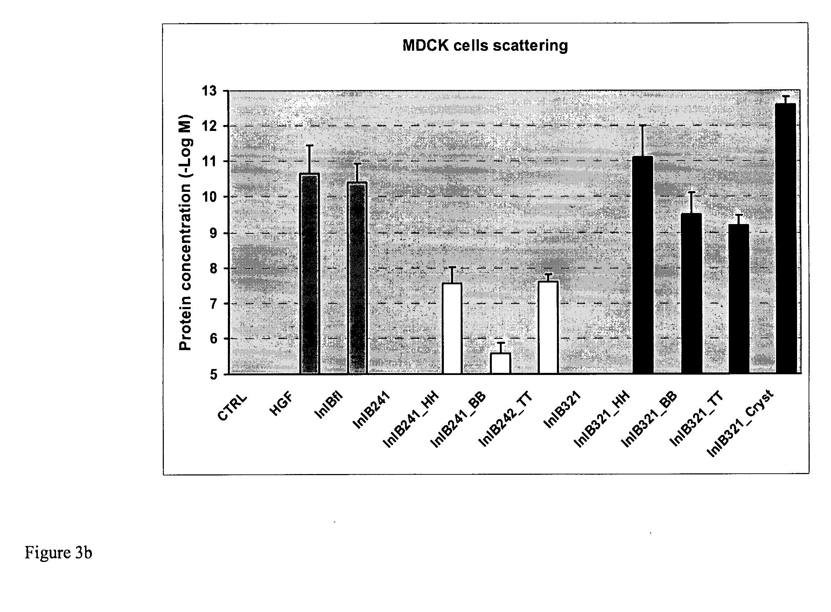

These results clearly show the different scattering potency of the monomeric and dimeric species of InIB₂₄₁ and InIB₃₂₁, see figure 3. While monomer of both InIB₂₄₁ and InIB₃₂₁ are not able to induce scattering even at relatively high concentrations, the addition of the dimeric species causes evident scattering of the cells. Moreover, the cell scattering capability is concentration-dependent and differs from dimer to dimer. The bar graph (Figure 3B) shows the overall results obtained from the experiment, with the x-axis indicating the various ligand species employed, and the y-axis the negative decimal logarithm of their minimal molar concentration required to induce cell scattering. The direct correlation between ligands concentrations and scattering potency is thus displayed in an intuitive way, i.e. the higher the bar, the higher the cell scattering potency.

According to these data, scattering potencies can be divided in three groups: the InIB₂₄₁ group (monomer and dimers), the InIB₃₂₁ group (monomer and dimers) and the InIB_{321_}Crys group.
- InIB₂₄₁ group (Cap domain + LRR domain): monomeric InIB₂₄₁ species doesn't cause any scattering of the cells, in line with what has been previously reported (Banerjee et al., 2004 supra; Niemann *et al.,* 2007 supra). Importantly, the Head-to-Head, Back-to-Back and Tail-to-tail dimers of InIB₂₄₁ cause evident cell scattering, although far weaker than HGF and InIB full length. Artificial ligand dimerization has therefore a strong effect on Met activation. Moreover, Head-to-Head and Tail-to-Tail dimers have the same scattering potency, while the ability of the Back-to-Back dimer in promoting cell scattering is low.
- InIB₃₂₁ group (Cap+LRR+IR domain): again, monomeric InIB₃₂₁ does not cause cell scattering, while all the other artificial dimers strongly promote it. Unlike InIB₂₄₁, the scattering potency of the InIB₃₂₁ dimers goes in the following order: InIB₃₂₁_HH > InIB_{321_}BB > InIB_{321_}TT.
- **InIB_{321_}Crys:** the ability of the "crystal contact" dimer to elicit cell scattering is extremely high and the "crystal contact" dimer is about two orders of magnitude more active than HGF and InIBfl.

### Example 3 Met and Erk1/2 phosphorylation

Phosphorylation of Met and of downstream signalling molecule Erk1/2 was assessed using starved HeLa cells stimulated for 5 minutes. Cells were subsequently lysed by addition of Crack Buffer. The lysate was run in an SDS-PAGE gel. Immunoblotting was performed using anti-phospho-Met and anti-phospho-Erk antibodies. Assay was performed in triplicate, and the reported blots are representative of the three experiments.

The Phosphorylation assay was performed as follows:

For this experiment, subconfluent HeLa cells were seeded at 5x10⁴ cells/plate either in a 6 well- or a 12 well-plate. Cells were starved overnight and medium was changed twice during starvation. The following day, cells were incubated with the protein solutions (2 ml for the 6 well plates, 1 ml for the 12 well plates) for 5 minutes, and the incubation was stopped by aspiring the medium and adding 60 ul of protein sample buffer containing 50 mM DTT. Cells were scraped and the lysate was loaded on a SDS-PAGE gel. Proteins were then blotted on a PVDF membrane subsequently blocked using a solution containing 5% BSA dissolved in TBST. Anti-phospho Met (α-p-Met Ab) and anti-phospho Erk1/2 antibodies (α-p-Erk Ab), purchased from Cell Signaling Technology (catalogue number 3126 and 9106, respectively), were dissolved in the blocking solution at a 1/1000 ratio for the a α-p-Met Ab and at a 1/2000 ratio for α-p-Met, added to the membrane and incubated overnight in the cold room. Rabbit (for α-p-Met Ab) and mouse (for α-p-Erk Ab) Ab were used as secondary Ab. These peroxidase-bound Ab were visualized on the membrane using an enhanced chemiluminescence (ECL) kit (Amersham Biosciences). Phosphorylated species were detected by western blot using a Fuji LAS 3000 imager.

Again, results can be divided in three distinct groups:
- **InIB₂₄₁ group:** monomeric InIB₂₄₁ does not promote Met and/or Erk1/2 phopshorylation, in agreement with the lack of cell scattering previously observed. Dimeric forms however show clear Met and Erk1/2 phosphorylation, in agreement with the cell scattering previously observed.
- **InIB₃₂₁ group:** unlike monomeric InIB₂₄₁, monomeric InIB₃₂₁ causes both Met and Erk1/2 phosphorylation, despite of its inability to cause cell scattering. This agrees with previous reports in the literature (Shen *et al.,* 2000 supra; Banerjee *et al.,* 2004 supra) and supports the suggested important role of the IR domain in triggering Met activation. As for dimers of InIB₂₄₁, dimers of InIB₃₂₁ are able to activate Met and Erk1/2 causing their phosphorylation. Among InIB₃₂₁ dimers, the "crystal dimer" shows a Met phosphorylation that is higher compared to the other InIB₃₂₁ dimers.
- **InIB₃₂₁ "crystal dimer":** compared to the other dimers included in the experiment, the InIB crystal dimer shows high activity towards Met phosphorylation. This agrees well with the high potency in the cell scattering assay. From this experiment, no phosphorylation of both Met and Erk1/2 is observed for concentrations of 10⁻¹⁰ M and below.

### Example 4 Elucidation of the InIB₃₂₁ "crystal dimer" structure

The nature of the InIB_{321_}Crys dimer itself and its subsequent unique potency in Met activation prompted us to solve the structure of this dimer with the aim of 1) verifying the effective reproduction of the crystal contact in solution and 2) gaining more insights on the structural reasons why this dimer is able to elicit such strong Met activation.

Initial crystals of InIB₃₂₁_Crys were grown from the same conditions as the rhombohedral crystal form of InIB₃₂₁, from which the crystal dimer was derived (40% PEG 300, 0.2 M NaCl, 0.1 M CHES, pH 9.5). These crystals suffered from severe intergrowth. Optimization of the crystallization conditions gave diffraction-quality crystals obtained by mixing the concentrated protein (6.7 mg/ml) in a 2:1 ratio with a reservoir solution containing 44-52% PEG 200, 0.1M CHES pH=9.5, 0.2M NaCl equilibrated against a reservoir volume of 70 µl. Crystals grew at 20°C in a few hours. Fully grown crystals were fished and flash frozen without the use of further cryoprotectants. The best crystal diffracted to 2.5 A. at beamline ID14-4 at the ESRF in Grenoble, and a dataset was taken. The structure of InIB₃₂₁ "crystal dimer" was solved by molecular replacement with the structure of InIB₃₂₁ as a search model (PDB code 1 H6T, Schubert *et al.,* 2001 supra).

The disulfide-linked crystal dimer crystallized in the same spacegroup (H32) and with the same cell constants as the rhombohedral crystal form of monomeric InIB₃₂₁. Accordingly, the crystal structure showed the two monomers of InIB bearing the expected "crystal dimer" arrangement. Electron density of the intermolecular disulfide bonds was clearly visible and the covalent bonds could be easily modelled.

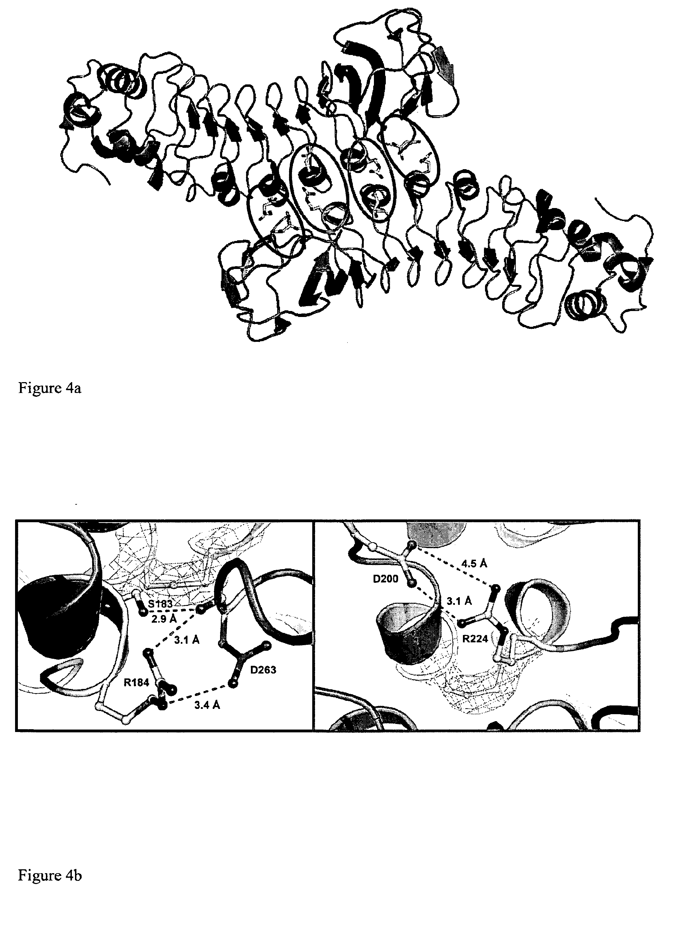

Figure 4 shows the model of the InIB crystal dimer according to the present invention. Beside the cysteine disulfide bridges, salt bridges between Arg 184 and Asp 263 and hydrogen bonds between Ser 183 and the oxygen of Asp 263 backbone contribute to the stabilization of the surface interaction. In addition, salt bridges between Asp 200 and Arg 224 are present. Figure 4a and Figure 4b show the positions of the residues involved and a detailed view of these interactions.

### Example 5 Stimulation of DNA synthesis in MK keratinocytes

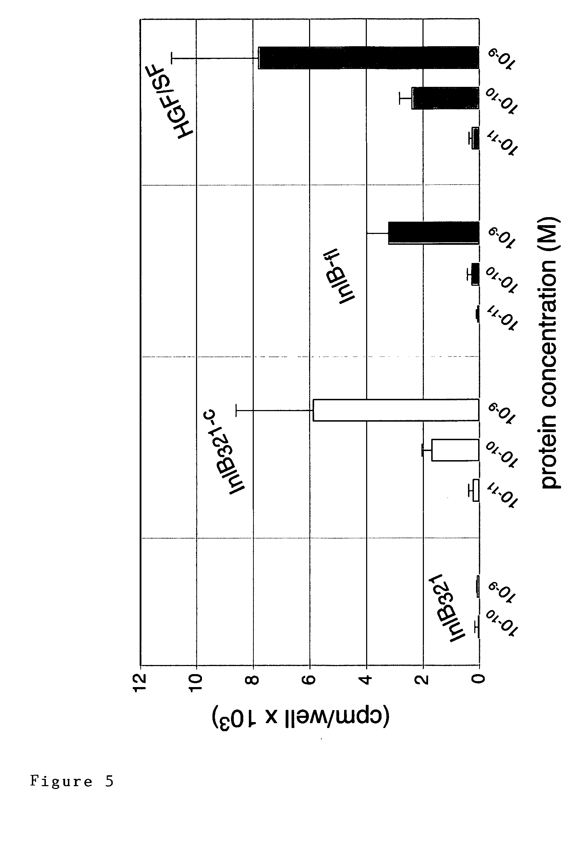

The ability of the InIB "crystal dimer" to stimulate cell division (its activity as a mitogen) tested by assaying the stimulation of DNA synthesis in the MK keratinocyte cell line. Monomeric InIB₃₂₁ was inactive at all concentrations tested. As shown in figure 5, full-length InIB stimulated DNA synthesis starting at a concentration of about 10⁻¹⁰ M. The InIB₃₂₁ "crystal dimer" was about 10 times more active than full-length InIB (stimulating DNA synthesis at a concentration of 10⁻¹¹ M and above). Although the activity of the InIB "crystal dimer" was lower than that of HGF/SF, this assay demonstrates that the InIB "crystal dimer" is a very potent agonist of the Met receptor.

The assay was carried out as described by Holmes (O. Holmes, *et al.* 2007, supra). Mouse keratinocyte (MK) cells were used to seed 24-well tissue culture plates (Falcon) at a density of 2500 cells per well. Cells were incubated in keratinocyte medium (Gibco) supplemented with 50 µg/ml bovine pituitary extract (BPE), 5 ng/ml recombinant epidermal growth factor (EGF) and penicillin/streptomycin (complete KM) at 37 °C/5% CO₂. When 100% confluence was reached, cells were washed twice with PBS, then incubated in serum-free keratinocyte medium (SFKM) for 24 h. SFKM (1 ml) containing 1 mg/ml BSA, 75 µg/ml thymidine, 1 µCi/ml [3H]methylthymidine was then added to each well followed by the addition of proteins diluted in SFKM. The cells were then incubated for 18 h to allow incorporation of [3H]methylthymidine into newly synthesised DNA. Free [3H]methylthymidine was removed by washing cells with 5% TCA, then cells were lysed with 0.2 M NaOH. Cell lysate was diluted 1:25 into scintillation fluid (Fluorosafe from BDH) and incubated at room temperature in the dark for 3 h before counting each vial for 1 min.

## Claims

1. Isolated, recombinant Internalin B (InIB) dimer consisting of two monomeric polypeptides being covalently linked with each other via at lest two bonds selected from disulfide bonds, direct bonds or other covalent linkages whereby said polypeptides are derived from the InIB protein and the bonds are located in the LRR domain corresponding to polypeptides of said protein of amino acids 81 to 241 of InIB of Seq. ID No. 1 wherein the monomeric polypeptides are arranged in an anti-parallel orientation.

2. The dimer according to claim 1, wherein at least two cysteines are present in the LRR of each monomer polypeptide introduced by genetic engineering.

3. The dimer according to claim 1 or 2, wherein any naturally occurring cysteine residue present in the LRR region of amino acids 81 to 241 of the InIB protein of Seq. ID. No. 1 has been mutated to any other amino acids, preferably to the amino acid alanine.

4. The dimer according any one of the preceding claims, wherein the monomeric polypeptides comprises aromatic amino acid residues at positions 104, 124, 170, and 214 of Seq. ID No. 1.

5. The dimer according to any one of the preceding claims, wherein in the monomeric polypeptide amino acid residues 206 and 227 of Seq. ID no. 1 have been mutated to cysteine and, optionally, amino acid residue 242 has been mutated to alanine.

6. The dimer according to any one of the preceding claims, wherein the monomeric polypeptide is a polypeptide of Seq. ID no. 2.

7. The dimer according to any one of the preceding claims, wherein the monomeric is the polypeptide of Seq. ID no. 3 (hier die genaue Sequenz InIB 36 bis 321 mutiert) and/or Seq. ID No. 4.

8. The dimer according to any one of the preceding claims, which is an agonist of the Met receptor.

9. The dimer according to any one of the preceding claims, wherein the monomeric polypeptide is
a) a peptide of Seq. ID No. 3 or 4,
b) a polypeptide comprising an amino acid sequence of Seq. ID No. 3 or 4 and exhibiting a binding to the Ig1 domain of the Met receptor;
c) a functional fragment or derivative of b) exhibiting binding to the Ig1 domain of the Met receptor;
d) a polypeptide having a 70 % or more homology with an amino acid sequence of the Seq. ID No. 3 or 4 or a functional fragment or derivative thereof whereby the cysteine residues in Seq. ID No. 3 or 4 and the aromatic amino acid residues at positions 104, 124, 170, and 214 of Seq. ID No. 1 remain unchanged;
e) an altered polypeptide in which at least parts of the amino acids have been replaced by analogous parts of a different specificity, in particular, human, while maintaining the ability to bind Ig1 domain of the Met receptor and to form dimers in an anti-parallel confirmation wherein the cysteine residues of Seq. ID Nos. 3 or 4 and the aromatic amino acid residues at position 104, 124 170, and 214 of Seq. ID Nos. 3 and 4 remain unchanged.

10. A nucleic acid molecule encoding a monomeric polypeptide as defined in any one of claims 1 to 9, in particular, a nucleic acid of Seq. ID No. 7.

11. Pharmaceutical composition comprising a dimer according to any one of claims 1 to 9 or the nucleic acid according to claim 10.

12. Pharmaceutical composition according to claim 11 for wound healing or tissue regeneration after tissue damage.

13. The pharmaceutical according to any one of the claims 10 or 11 for treatment of tissue damages, in particular, skin or mucosal tissue damages, in particular, due in the course of progressing diabetes.

14. Pharmaceutical composition according to any one of claims 10 to 12 for the prevention or treatment of liver injuries, in particular, liver repair, lung injuries, kidney injuries, and heart muscle repair.

15. The use of a dimer according to any one of claims 1 to 9 as an *in vitro* stimulator of cell proliferation, in particular, in tissue engineering techniques.


SEQUENCE LISTING

<110>  Helmholtz-Zentrum für Infektionsforschung

<120>  InlB dimer

<130>  3275020

<160>  7

<170>  PatentIn version 3.3

<210>  1
<211>  630
<212>  PRT
<213>  l. monocytogenes

<400>  1

Met Lys Glu Lys His Asn Pro Arg Arg Lys Tyr Cys Leu Ile Ser Gly
1               5                   10                  15

Leu Ala Ile Ile Phe Ser Leu Trp Ile Ile Ile Gly Asn Gly Ala Lys
            20                  25                  30

Val Gln Ala Glu Thr Ile Thr Val Pro Thr Pro Ile Lys Gln Ile Phe
        35                  40                  45

Ser Asp Asp Ala Phe Ala Glu Thr Ile Lys Asp Asn Leu Lys Lys Lys
    50                  55                  60

Ser Val Thr Asp Ala Val Thr Gln Asn Glu Leu Asn Ser Ile Asp Gln
65                  70                  75                  80

Ile Ile Ala Asn Asn Ser Asp Ile Lys Ser Val Gln Gly Ile Gln Tyr
                85                  90                  95

Leu Pro Asn Val Thr Lys Leu Phe Leu Asn Gly Asn Lys Leu Thr Asp
            100                 105                 110

Ile Lys Pro Leu Ala Asn Leu Lys Asn Leu Gly Trp Leu Phe Leu Asp
        115                 120                 125

Glu Asn Lys Val Lys Asp Leu Ser Ser Leu Lys Asp Leu Lys Lys Leu
    130                 135                 140

Lys Ser Leu Ser Leu Glu His Asn Gly Ile Ser Asp Ile Asn Gly Leu
145                 150                 155                 160

Val His Leu Pro Gln Leu Glu Ser Leu Tyr Leu Gly Asn Asn Lys Ile
                165                 170                 175

Thr Asp Ile Thr Val Leu Ser Arg Leu Thr Lys Leu Asp Thr Leu Ser
            180                 185                 190

Leu Glu Asp Asn Gln Ile Ser Asp Ile Val Pro Leu Ala Gly Leu Thr

        195                 200                 205

Lys Leu Gln Asn Leu Tyr Leu Ser Lys Asn His Ile Ser Asp Leu Arg
    210                 215                 220

Ala Leu Ala Gly Leu Lys Asn Leu Asp Val Leu Glu Leu Phe Ser Gln
225                 230                 235                 240

Glu Cys Leu Asn Lys Pro Ile Asn His Gln Ser Asn Leu Val Val Pro
                245                 250                 255

Asn Thr Val Lys Asn Thr Asp Gly Ser Leu Val Thr Pro Glu Ile Ile
            260                 265                 270

Ser Asp Asp Gly Asp Tyr Glu Lys Pro Asn Val Lys Trp His Leu Pro
        275                 280                 285

Glu Phe Thr Asn Glu Val Ser Phe Ile Phe Tyr Gln Pro Val Thr Ile
    290                 295                 300

Gly Lys Ala Lys Ala Arg Phe His Gly Arg Val Thr Gln Pro Leu Lys
305                 310                 315                 320

Glu Val Tyr Thr Val Ser Tyr Asp Val Asp Gly Thr Val Ile Lys Thr
                325                 330                 335

Lys Val Glu Ala Gly Thr Arg Ile Thr Ala Pro Lys Pro Pro Thr Lys
            340                 345                 350

Gln Gly Tyr Val Phe Lys Gly Trp Tyr Thr Glu Lys Asn Gly Gly His
        355                 360                 365

Glu Trp Asn Phe Asn Thr Asp Tyr Met Ser Gly Asn Asp Phe Thr Leu
    370                 375                 380

Tyr Ala Val Phe Lys Ala Glu Thr Thr Glu Lys Ala Val Asn Leu Thr
385                 390                 395                 400

Arg Tyr Val Lys Tyr Ile Arg Gly Asn Ala Gly Ile Tyr Lys Leu Pro
                405                 410                 415

Arg Glu Asp Asn Ser Leu Lys Gln Gly Thr Leu Ala Ser His Arg Cys
            420                 425                 430

Lys Ala Leu Thr Val Asp Arg Glu Ala Arg Asn Gly Gly Lys Leu Trp
        435                 440                 445

Tyr Arg Leu Lys Asn Ile Gly Trp Thr Lys Ala Glu Asn Leu Ser Leu
    450                 455                 460

Asp Arg Tyr Asp Lys Met Glu Tyr Asp Lys Gly Val Thr Ala Tyr Ala

465                 470                 475                 480

Arg Val Arg Asn Ala Ser Gly Asn Ser Val Trp Thr Lys Pro Tyr Asn
                485                 490                 495

Thr Ala Gly Ala Lys His Val Asn Lys Leu Ser Val Tyr Gln Gly Lys
            500                 505                 510

Asn Met Arg Ile Leu Arg Glu Ala Lys Thr Pro Ile Thr Thr Trp Tyr
        515                 520                 525

Gln Phe Ser Ile Gly Gly Lys Val Ile Gly Trp Val Asp Thr Arg Ala
    530                 535                 540

Leu Asn Thr Phe Tyr Lys Gln Ser Met Glu Lys Pro Thr Arg Leu Thr
545                 550                 555                 560

Arg Tyr Val Ser Ala Asn Lys Ala Gly Glu Ser Tyr Tyr Lys Val Pro
                565                 570                 575

Val Ala Asp Asn Pro Val Lys Arg Gly Thr Leu Ala Lys Tyr Lys Asn
            580                 585                 590

Gln Lys Leu Ile Val Asp Cys Gln Ala Thr Ile Glu Gly Gln Leu Trp
        595                 600                 605

Tyr Arg Ile Arg Thr Ser Ser Thr Phe Ile Gly Trp Thr Lys Ala Ala
    610                 615                 620

Asn Leu Arg Ala Gln Lys
625                 630

<210>  2
<211>  124
<212>  PRT
<213>  artificial

<220>
<223>  minimal sequence containing cysteine residues and aromatic
       residues

<220>
<221>  MISC_FEATURE
<222>  (2)..(20)
<223>  Xaa can be any naturally occuring amino acid other than Cys

<220>
<221>  MISC_FEATURE
<222>  (22)..(66)
<223>  Xaa can be any naturally occuring amino acid other than Cys

<220>
<221>  MISC_FEATURE
<222>  (68)..(102)
<223>  Xaa can be any naturally occuring amino acid other than Cys

<220>
<221>  MISC_FEATURE
<222>  (104)..(110)
<223>  Xaa can be any naturally occuring amino acid other than Cys

<220>
<221>  MISC_FEATURE
<222>  (112)..(123)
<223>  Xaa can be any naturally occuring amino acid other than Cys

<400>  2

Phe Xaa Xaa Xaa Xaa Xaa Xaa Xaa Xaa Xaa Xaa Xaa Xaa Xaa Xaa Xaa
1               5                   10                  15

Xaa Xaa Xaa Xaa Trp Xaa Xaa Xaa Xaa Xaa Xaa Xaa Xaa Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Tyr Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Xaa Xaa Xaa
                85                  90                  95

Xaa Xaa Xaa Xaa Xaa Xaa Cys Xaa Xaa Xaa Xaa Xaa Xaa Xaa Tyr Xaa
            100                 105                 110

Xaa Xaa Xaa Xaa Xaa Xaa Xaa Xaa Xaa Xaa Xaa Cys
        115                 120


<210>  3
<211>  285
<212>  PRT
<213>  artificial

<220>
<223>  INlb 321 mutated with G206C and A227C

<400>  3

Glu Thr Ile Thr Val Pro Thr Pro Ile Lys Gln Ile Phe Ser Asp Asp
1               5                   10                  15

Ala Phe Ala Glu Thr Ile Lys Asp Asn Leu Lys Lys Lys Ser Val Thr
            20                  25                  30

Asp Ala Val Thr Gln Asn Glu Leu Asn Ser Ile Asp Gln Ile Ile Ala
        35                  40                  45

Asn Asn Ser Asp Ile Lys Ser Val Gln Gly Ile Gln Tyr Leu Pro Asn
    50                  55                  60

Val Thr Lys Leu Phe Leu Asn Gly Asn Lys Leu Thr Asp Ile Lys Pro
65                  70                  75                  80

Leu Ala Asn Leu Lys Asn Leu Gly Trp Leu Phe Leu Asp Glu Asn Lys
                85                  90                  95

Val Lys Asp Leu Ser Ser Leu Lys Asp Leu Lys Lys Leu Lys Ser Leu
            100                 105                 110

Ser Leu Glu His Asn Gly Ile Ser Asp Ile Asn Gly Leu Val His Leu
        115                 120                 125

Pro Gln Leu Glu Ser Leu Tyr Leu Gly Asn Asn Lys Ile Thr Asp Ile
    130                 135                 140

Thr Val Leu Ser Arg Leu Thr Lys Leu Asp Thr Leu Ser Leu Glu Asp
145                 150                 155                 160

Asn Gln Ile Ser Asp Ile Val Pro Leu Ala Cys Leu Thr Lys Leu Gln
                165                 170                 175

Asn Leu Tyr Leu Ser Lys Asn His Ile Ser Asp Leu Arg Ala Leu Cys
            180                 185                 190

Gly Leu Lys Asn Leu Asp Val Leu Glu Leu Phe Ser Gln Glu Cys Leu
        195                 200                 205

Asn Lys Pro Ile Asn His Gln Ser Asn Leu Val Val Pro Asn Thr Val
    210                 215                 220

Lys Asn Thr Asp Gly Ser Leu Val Thr Pro Glu Ile Ile Ser Asp Asp
225                 230                 235                 240

Gly Asp Tyr Glu Lys Pro Asn Val Lys Trp His Leu Pro Glu Phe Thr
                245                 250                 255

Asn Glu Val Ser Phe Ile Phe Tyr Gln Pro Val Thr Ile Gly Lys Ala
            260                 265                 270

Lys Ala Arg Phe His Gly Arg Val Thr Gln Pro Leu Lys
        275                 280                 285

<210>  4
<211>  285
<212>  PRT
<213>  artificial

<220>
<223>  InlB with C242A G206C and A227C

<400>  4

Glu Thr Ile Thr Val Pro Thr Pro Ile Lys Gln Ile Phe Ser Asp Asp
1               5                   10                  15

Ala Phe Ala Glu Thr Ile Lys Asp Asn Leu Lys Lys Lys Ser Val Thr
            20                  25                  30

Asp Ala Val Thr Gln Asn Glu Leu Asn Ser Ile Asp Gln Ile Ile Ala
        35                  40                  45

Asn Asn Ser Asp Ile Lys Ser Val Gln Gly Ile Gln Tyr Leu Pro Asn
    50                  55                  60

Val Thr Lys Leu Phe Leu Asn Gly Asn Lys Leu Thr Asp Ile Lys Pro
65                  70                  75                  80

Leu Ala Asn Leu Lys Asn Leu Gly Trp Leu Phe Leu Asp Glu Asn Lys
                85                  90                  95

Val Lys Asp Leu Ser Ser Leu Lys Asp Leu Lys Lys Leu Lys Ser Leu
            100                 105                 110

Ser Leu Glu His Asn Gly Ile Ser Asp Ile Asn Gly Leu Val His Leu
        115                 120                 125

Pro Gln Leu Glu Ser Leu Tyr Leu Gly Asn Asn Lys Ile Thr Asp Ile
    130                 135                 140

Thr Val Leu Ser Arg Leu Thr Lys Leu Asp Thr Leu Ser Leu Glu Asp
145                 150                 155                 160

Asn Gln Ile Ser Asp Ile Val Pro Leu Ala Cys Leu Thr Lys Leu Gln
                165                 170                 175

Asn Leu Tyr Leu Ser Lys Asn His Ile Ser Asp Leu Arg Ala Leu Cys
            180                 185                 190

Gly Leu Lys Asn Leu Asp Val Leu Glu Leu Phe Ser Gln Glu Ala Leu
        195                 200                 205

Asn Lys Pro Ile Asn His Gln Ser Asn Leu Val Val Pro Asn Thr Val
    210                 215                 220

Lys Asn Thr Asp Gly Ser Leu Val Thr Pro Glu Ile Ile Ser Asp Asp
225                 230                 235                 240

Gly Asp Tyr Glu Lys Pro Asn Val Lys Trp His Leu Pro Glu Phe Thr
                245                 250                 255

Asn Glu Val Ser Phe Ile Phe Tyr Gln Pro Val Thr Ile Gly Lys Ala
            260                 265                 270

Lys Ala Arg Phe His Gly Arg Val Thr Gln Pro Leu Lys
        275                 280                 285

<210>  5
<211>  285
<212>  PRT
<213>  L. monocytogenes

<400>  5

Glu Thr Ile Thr Val Pro Thr Pro Ile Lys Gln Ile Phe Ser Asp Asp
1               5                   10                  15

Ala Phe Ala Glu Thr Ile Lys Asp Asn Leu Lys Lys Lys Ser Val Thr
            20                  25                  30

Asp Ala Val Thr Gln Asn Glu Leu Asn Ser Ile Asp Gln Ile Ile Ala
        35                  40                  45

Asn Asn Ser Asp Ile Lys Ser Val Gln Gly Ile Gln Tyr Leu Pro Asn
    50                  55                  60

Val Thr Lys Leu Phe Leu Asn Gly Asn Lys Leu Thr Asp Ile Lys Pro
65                  70                  75                  80

Leu Ala Asn Leu Lys Asn Leu Gly Trp Leu Phe Leu Asp Glu Asn Lys
                85                  90                  95

Val Lys Asp Leu Ser Ser Leu Lys Asp Leu Lys Lys Leu Lys Ser Leu
            100                 105                 110

Ser Leu Glu His Asn Gly Ile Ser Asp Ile Asn Gly Leu Val His Leu
        115                 120                 125

Pro Gln Leu Glu Ser Leu Tyr Leu Gly Asn Asn Lys Ile Thr Asp Ile
    130                 135                 140

Thr Val Leu Ser Arg Leu Thr Lys Leu Asp Thr Leu Ser Leu Glu Asp
145                 150                 155                 160

Asn Gln Ile Ser Asp Ile Val Pro Leu Ala Gly Leu Thr Lys Leu Gln
                165                 170                 175

Asn Leu Tyr Leu Ser Lys Asn His Ile Ser Asp Leu Arg Ala Leu Ala
            180                 185                 190

Gly Leu Lys Asn Leu Asp Val Leu Glu Leu Phe Ser Gln Glu Cys Leu
        195                 200                 205

Asn Lys Pro Ile Asn His Gln Ser Asn Leu Val Val Pro Asn Thr Val
    210                 215                 220

Lys Asn Thr Asp Gly Ser Leu Val Thr Pro Glu Ile Ile Ser Asp Asp

225                 230                 235                 240

Gly Asp Tyr Glu Lys Pro Asn Val Lys Trp His Leu Pro Glu Phe Thr
                245                 250                 255

Asn Glu Val Ser Phe Ile Phe Tyr Gln Pro Val Thr Ile Gly Lys Ala
            260                 265                 270

Lys Ala Arg Phe His Gly Arg Val Thr Gln Pro Leu Lys
        275                 280                 285

<210>  6
<211>  855
<212>  DNA
<213>  L. monocytogenes

<400>  6
gagactatca ccgtgccaac gccaatcaag caaatttttt cagatgatgc ttttgcagaa      60

acaatcaaag acaatttaaa gaaaaaaagt gtgacagatg cagtgacaca aaatgaatta     120

aatagtatag atcaaatcat tgcgaataat agtgatatta aatccgttca aggaattcag     180

tatttaccca atgtgacaaa gttattttta aacgggaata aactaacaga tataaagccc     240

ttagcaaact tgaaaaattt aggatggctt tttttagacg aaaataaagt taaggaccta     300

agttcgctca aggatttaaa aaaattaaaa tcactttctt tggagcataa tggtataagt     360

gatataaacg gacttgttca tttaccacag ctggaaagtt tgtatttggg aaataataaa     420

ataacggata taacggttct ttcacgttta actaaactgg atactttgtc tctcgaagat     480

aaccaaatta gtgatattgt gccacttgca ggtttaacta aattgcagaa cctatattta     540

agtaaaaatc acataagcga tttaagagca ttagcaggac ttaaaaatct agatgtttta     600

gaattattta gccaagaatg tcttaataag cctattaatc atcaatctaa tttggttgtt     660

ccgaatacag tgaaaaacac tgatgggtcg ttagtgactc cagaaataat aagtgatgat     720

ggcgattatg aaaaacctaa tgttaaatgg catttaccag aatttacaaa tgaagtgagt     780

tttattttct atcagccagt cactattgga aaagcaaaag caagatttca tgggagagta     840

acccaaccac tgaaa                                                      855

<210>  7
<211>  855
<212>  DNA
<213>  artificial

<220>
<223>  InlB DNA mutated to achieve C242A G206C A227C

<400>  7
gagactatca ccgtgccaac gccaatcaag caaatttttt cagatgatgc ttttgcagaa      60

acaatcaaag acaatttaaa gaaaaaaagt gtgacagatg cagtgacaca aaatgaatta     120

aatagtatag atcaaatcat tgcgaataat agtgatatta aatccgttca aggaattcag     180

tatttaccca atgtgacaaa gttattttta aacgggaata aactaacaga tataaagccc     240

ttagcaaact tgaaaaattt aggatggctt tttttagacg aaaataaagt taaggaccta 300
agttcgctca aggatttaaa aaaattaaaa tcactttctt tggagcataa tggtataagt 360
gatataaacg gacttgttca tttaccacag ctggaaagtt tgtatttggg aaataataaa 420
ataacggata taacggttct ttcacgttta actaaactgg atactttgtc tctcgaagat 480
aaccaaatta gtgatattgt gccacttgca tgtttaacta aattgcagaa cctatattta 540
agtaaaaatc acataagcga tttaagagca ttatgcggac ttaaaaatct agatgtttta 600
gaattattta gccaagaagc tcttaataag cctattaatc atcaatctaa tttggttgtt 660
ccgaatacag tgaaaaacac tgatgggtcg ttagtgactc cagaaataat aagtgatgat 720
ggcgattatg aaaaacctaa tgttaaatgg catttaccag aatttacaaa tgaagtgagt 780
tttattttct atcagccagt cactattgga aaagcaaaag caagatttca tgggagagta 840
acccaaccac tgaaa 855

SEQUENCE LISTING

<110> Helmholtz-Zentrum für Infektionsforschung

<120> InlB dimer

<130> 3275020

<160> 7

<170> PatentIn version 3.3

<210> 1
<211> 630
<212> PRT
<213> l. monocytogenes

<400> 1

Met Lys Glu Lys His Asn Pro Arg Arg Lys Tyr Cys Leu Ile Ser Gly
1               5                   10                  15

Leu Ala Ile Ile Phe Ser Leu Trp Ile Ile Ile Gly Asn Gly Ala Lys
            20                  25                  30

Val Gln Ala Glu Thr Ile Thr Val Pro Thr Pro Ile Lys Gln Ile Phe
        35                  40                  45

Ser Asp Asp Ala Phe Ala Glu Thr Ile Lys Asp Asn Leu Lys Lys Lys
    50                  55                  60

Ser Val Thr Asp Ala Val Thr Gln Asn Glu Leu Asn Ser Ile Asp Gln
65                  70                  75                  80

Ile Ile Ala Asn Asn Ser Asp Ile Lys Ser Val Gln Gly Ile Gln Tyr
                85                  90                  95

Leu Pro Asn Val Thr Lys Leu Phe Leu Asn Gly Asn Lys Leu Thr Asp
            100                 105                 110

Ile Lys Pro Leu Ala Asn Leu Lys Asn Leu Gly Trp Leu Phe Leu Asp
        115                 120                 125

Glu Asn Lys Val Lys Asp Leu Ser Ser Leu Lys Asp Leu Lys Lys Leu
    130                 135                 140

Lys Ser Leu Ser Leu Glu His Asn Gly Ile Ser Asp Ile Asn Gly Leu
145                 150                 155                 160

Val His Leu Pro Gln Leu Glu Ser Leu Tyr Leu Gly Asn Asn Lys Ile
                165                 170                 175

Thr Asp Ile Thr Val Leu Ser Arg Leu Thr Lys Leu Asp Thr Leu Ser
            180                 185                 190

Leu Glu Asp Asn Gln Ile Ser Asp Ile Val Pro Leu Ala Gly Leu Thr

        195                 200                 205

Lys Leu Gln Asn Leu Tyr Leu Ser Lys Asn His Ile Ser Asp Leu Arg
    210                 215                 220

Ala Leu Ala Gly Leu Lys Asn Leu Asp Val Leu Glu Leu Phe Ser Gln
225                 230                 235                 240

Glu Cys Leu Asn Lys Pro Ile Asn His Gln Ser Asn Leu Val Val Pro
                245                 250                 255

Asn Thr Val Lys Asn Thr Asp Gly Ser Leu Val Thr Pro Glu Ile Ile
            260                 265                 270

Ser Asp Asp Gly Asp Tyr Glu Lys Pro Asn Val Lys Trp His Leu Pro
        275                 280                 285

Glu Phe Thr Asn Glu Val Ser Phe Ile Phe Tyr Gln Pro Val Thr Ile
    290                 295                 300

Gly Lys Ala Lys Ala Arg Phe His Gly Arg Val Thr Gln Pro Leu Lys
305                 310                 315                 320

Glu Val Tyr Thr Val Ser Tyr Asp Val Asp Gly Thr Val Ile Lys Thr
                325                 330                 335

Lys Val Glu Ala Gly Thr Arg Ile Thr Ala Pro Lys Pro Pro Thr Lys
            340                 345                 350

Gln Gly Tyr Val Phe Lys Gly Trp Tyr Thr Glu Lys Asn Gly Gly His
        355                 360                 365

Glu Trp Asn Phe Asn Thr Asp Tyr Met Ser Gly Asn Asp Phe Thr Leu
    370                 375                 380

Tyr Ala Val Phe Lys Ala Glu Thr Thr Glu Lys Ala Val Asn Leu Thr
385                 390                 395                 400

Arg Tyr Val Lys Tyr Ile Arg Gly Asn Ala Gly Ile Tyr Lys Leu Pro
                405                 410                 415

Arg Glu Asp Asn Ser Leu Lys Gln Gly Thr Leu Ala Ser His Arg Cys
            420                 425                 430

Lys Ala Leu Thr Val Asp Arg Glu Ala Arg Asn Gly Gly Lys Leu Trp
        435                 440                 445

Tyr Arg Leu Lys Asn Ile Gly Trp Thr Lys Ala Glu Asn Leu Ser Leu
    450                 455                 460

Asp Arg Tyr Asp Lys Met Glu Tyr Asp Lys Gly Val Thr Ala Tyr Ala

465                 470                 475                 480

Arg Val Arg Asn Ala Ser Gly Asn Ser Val Trp Thr Lys Pro Tyr Asn
                485                 490                 495

Thr Ala Gly Ala Lys His Val Asn Lys Leu Ser Val Tyr Gln Gly Lys
            500                 505                 510

Asn Met Arg Ile Leu Arg Glu Ala Lys Thr Pro Ile Thr Thr Trp Tyr
        515                 520                 525

Gln Phe Ser Ile Gly Gly Lys Val Ile Gly Trp Val Asp Thr Arg Ala
    530                 535                 540

Leu Asn Thr Phe Tyr Lys Gln Ser Met Glu Lys Pro Thr Arg Leu Thr
545                 550                 555                 560

Arg Tyr Val Ser Ala Asn Lys Ala Gly Glu Ser Tyr Tyr Lys Val Pro
                565                 570                 575

Val Ala Asp Asn Pro Val Lys Arg Gly Thr Leu Ala Lys Tyr Lys Asn
            580                 585                 590

Gln Lys Leu Ile Val Asp Cys Gln Ala Thr Ile Glu Gly Gln Leu Trp
        595                 600                 605

Tyr Arg Ile Arg Thr Ser Ser Thr Phe Ile Gly Trp Thr Lys Ala Ala
    610                 615                 620

Asn Leu Arg Ala Gln Lys
625                 630

<210>  2
<211>  124
<212>  PRT
<213>  artificial

<220>
<223>  minimal sequence containing cysteine residues and aromatic
       residues

<220>
<221>  MISC_FEATURE
<222>  (2)..(20)
<223>  Xaa can be any naturally occuring amino acid other than Cys

<220>
<221>  MISC_FEATURE
<222>  (22)..(66)
<223>  Xaa can be any naturally occuring amino acid other than Cys

<220>
<221>  MISC_FEATURE
<222>  (68)..(102)
<223>  Xaa can be any naturally occuring amino acid other than Cys

<220>
<221>  MISC_FEATURE
<222>  (104)..(110)
<223>  Xaa can be any naturally occuring amino acid other than Cys

<220>
<221>  MISC_FEATURE
<222>  (112)..(123)
<223>  Xaa can be any naturally occuring amino acid other than Cys

<400>  2

Phe Xaa Xaa Xaa Xaa Xaa Xaa Xaa Xaa Xaa Xaa Xaa Xaa Xaa Xaa Xaa
1               5                   10                  15

Xaa Xaa Xaa Xaa Trp Xaa Xaa Xaa Xaa Xaa Xaa Xaa Xaa Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Tyr Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Xaa Xaa Xaa
                85                  90                  95

Xaa Xaa Xaa Xaa Xaa Xaa Cys Xaa Xaa Xaa Xaa Xaa Xaa Xaa Tyr Xaa
            100                 105                 110

Xaa Xaa Xaa Xaa Xaa Xaa Xaa Xaa Xaa Xaa Xaa Cys
        115                 120

<210>  3
<211>  285
<212>  PRT
<213>  artificial

<220>
<223>  INlb 321 mutated with G206C and A227C

<400>  3

Glu Thr Ile Thr Val Pro Thr Pro Ile Lys Gln Ile Phe Ser Asp Asp
1               5                   10                  15

Ala Phe Ala Glu Thr Ile Lys Asp Asn Leu Lys Lys Lys Ser Val Thr
            20                  25                  30

Asp Ala Val Thr Gln Asn Glu Leu Asn Ser Ile Asp Gln Ile Ile Ala
        35                  40                  45

Asn Asn Ser Asp Ile Lys Ser Val Gln Gly Ile Gln Tyr Leu Pro Asn
    50                  55                  60

Val Thr Lys Leu Phe Leu Asn Gly Asn Lys Leu Thr Asp Ile Lys Pro
65                  70                  75                  80

Leu Ala Asn Leu Lys Asn Leu Gly Trp Leu Phe Leu Asp Glu Asn Lys
                85                  90                  95

Val Lys Asp Leu Ser Ser Leu Lys Asp Leu Lys Lys Leu Lys Ser Leu
            100                 105                 110

Ser Leu Glu His Asn Gly Ile Ser Asp Ile Asn Gly Leu Val His Leu
        115                 120                 125

Pro Gln Leu Glu Ser Leu Tyr Leu Gly Asn Asn Lys Ile Thr Asp Ile
    130                 135                 140

Thr Val Leu Ser Arg Leu Thr Lys Leu Asp Thr Leu Ser Leu Glu Asp
145                 150                 155                 160

Asn Gln Ile Ser Asp Ile Val Pro Leu Ala Cys Leu Thr Lys Leu Gln
                165                 170                 175

Asn Leu Tyr Leu Ser Lys Asn His Ile Ser Asp Leu Arg Ala Leu Cys
            180                 185                 190

Gly Leu Lys Asn Leu Asp Val Leu Glu Leu Phe Ser Gln Glu Cys Leu
        195                 200                 205

Asn Lys Pro Ile Asn His Gln Ser Asn Leu Val Val Pro Asn Thr Val
    210                 215                 220

Lys Asn Thr Asp Gly Ser Leu Val Thr Pro Glu Ile Ile Ser Asp Asp
225                 230                 235                 240

Gly Asp Tyr Glu Lys Pro Asn Val Lys Trp His Leu Pro Glu Phe Thr
                245                 250                 255

Asn Glu Val Ser Phe Ile Phe Tyr Gln Pro Val Thr Ile Gly Lys Ala
            260                 265                 270

Lys Ala Arg Phe His Gly Arg Val Thr Gln Pro Leu Lys
        275                 280                 285

<210>  4
<211>  285
<212>  PRT
<213>  artificial

<220>
<223>  InlB with C242A G206C and A227C

<400>  4

Glu Thr Ile Thr Val Pro Thr Pro Ile Lys Gln Ile Phe Ser Asp Asp
1               5                   10                  15

Ala Phe Ala Glu Thr Ile Lys Asp Asn Leu Lys Lys Lys Ser Val Thr
            20                  25                  30

Asp Ala Val Thr Gln Asn Glu Leu Asn Ser Ile Asp Gln Ile Ile Ala
        35                  40                  45

Asn Asn Ser Asp Ile Lys Ser Val Gln Gly Ile Gln Tyr Leu Pro Asn
    50                  55                  60

Val Thr Lys Leu Phe Leu Asn Gly Asn Lys Leu Thr Asp Ile Lys Pro
65                  70                  75                  80

Leu Ala Asn Leu Lys Asn Leu Gly Trp Leu Phe Leu Asp Glu Asn Lys
                85                  90                  95

Val Lys Asp Leu Ser Ser Leu Lys Asp Leu Lys Lys Leu Lys Ser Leu
            100                 105                 110

Ser Leu Glu His Asn Gly Ile Ser Asp Ile Asn Gly Leu Val His Leu
        115                 120                 125

Pro Gln Leu Glu Ser Leu Tyr Leu Gly Asn Asn Lys Ile Thr Asp Ile
    130                 135                 140

Thr Val Leu Ser Arg Leu Thr Lys Leu Asp Thr Leu Ser Leu Glu Asp
145                 150                 155                 160

Asn Gln Ile Ser Asp Ile Val Pro Leu Ala Cys Leu Thr Lys Leu Gln
                165                 170                 175

Asn Leu Tyr Leu Ser Lys Asn His Ile Ser Asp Leu Arg Ala Leu Cys
            180                 185                 190

Gly Leu Lys Asn Leu Asp Val Leu Glu Leu Phe Ser Gln Glu Ala Leu
        195                 200                 205

Asn Lys Pro Ile Asn His Gln Ser Asn Leu Val Val Pro Asn Thr Val
    210                 215                 220

Lys Asn Thr Asp Gly Ser Leu Val Thr Pro Glu Ile Ile Ser Asp Asp
225                 230                 235                 240

Gly Asp Tyr Glu Lys Pro Asn Val Lys Trp His Leu Pro Glu Phe Thr
                245                 250                 255

Asn Glu Val Ser Phe Ile Phe Tyr Gln Pro Val Thr Ile Gly Lys Ala
            260                 265                 270

Lys Ala Arg Phe His Gly Arg Val Thr Gln Pro Leu Lys
        275                 280                 285

<210>  5
<211>  285
<212>  PRT
<213>  L. monocytogenes

<400>  5

Glu Thr Ile Thr Val Pro Thr Pro Ile Lys Gln Ile Phe Ser Asp Asp
1               5                   10                  15

Ala Phe Ala Glu Thr Ile Lys Asp Asn Leu Lys Lys Lys Ser Val Thr
            20                  25                  30

Asp Ala Val Thr Gln Asn Glu Leu Asn Ser Ile Asp Gln Ile Ile Ala
        35                  40                  45

Asn Asn Ser Asp Ile Lys Ser Val Gln Gly Ile Gln Tyr Leu Pro Asn
    50                  55                  60

Val Thr Lys Leu Phe Leu Asn Gly Asn Lys Leu Thr Asp Ile Lys Pro
65                  70                  75                  80

Leu Ala Asn Leu Lys Asn Leu Gly Trp Leu Phe Leu Asp Glu Asn Lys
                85                  90                  95

Val Lys Asp Leu Ser Ser Leu Lys Asp Leu Lys Lys Leu Lys Ser Leu
            100                 105                 110

Ser Leu Glu His Asn Gly Ile Ser Asp Ile Asn Gly Leu Val His Leu
        115                 120                 125

Pro Gln Leu Glu Ser Leu Tyr Leu Gly Asn Asn Lys Ile Thr Asp Ile
    130                 135                 140

Thr Val Leu Ser Arg Leu Thr Lys Leu Asp Thr Leu Ser Leu Glu Asp
145                 150                 155                 160

Asn Gln Ile Ser Asp Ile Val Pro Leu Ala Gly Leu Thr Lys Leu Gln
                165                 170                 175

Asn Leu Tyr Leu Ser Lys Asn His Ile Ser Asp Leu Arg Ala Leu Ala
            180                 185                 190

Gly Leu Lys Asn Leu Asp Val Leu Glu Leu Phe Ser Gln Glu Cys Leu
        195                 200                 205

Asn Lys Pro Ile Asn His Gln Ser Asn Leu Val Val Pro Asn Thr Val
    210                 215                 220

Lys Asn Thr Asp Gly Ser Leu Val Thr Pro Glu Ile Ile Ser Asp Asp

225                 230                 235                 240

Gly Asp Tyr Glu Lys Pro Asn Val Lys Trp His Leu Pro Glu Phe Thr
                245                 250                 255

Asn Glu Val Ser Phe Ile Phe Tyr Gln Pro Val Thr Ile Gly Lys Ala
            260                 265                 270

Lys Ala Arg Phe His Gly Arg Val Thr Gln Pro Leu Lys
        275                 280                 285

<210>  6
<211>  855
<212>  DNA
<213>  L. monocytogenes

<400>  6
gagactatca ccgtgccaac gccaatcaag caaatttttt cagatgatgc ttttgcagaa      60

acaatcaaag acaatttaaa gaaaaaaagt gtgacagatg cagtgacaca aaatgaatta     120

aatagtatag atcaaatcat tgcgaataat agtgatatta aatccgttca aggaattcag     180

tatttaccca atgtgacaaa gttattttta aacgggaata aactaacaga tataaagccc     240

ttagcaaact tgaaaaattt aggatggctt tttttagacg aaaataaagt taaggaccta     300

agttcgctca aggatttaaa aaaattaaaa tcactttctt tggagcataa tggtataagt     360

gatataaacg gacttgttca tttaccacag ctggaaagtt tgtatttggg aaataataaa     420

ataacggata taacggttct ttcacgttta actaaactgg atactttgtc tctcgaagat     480

aaccaaatta gtgatattgt gccacttgca ggtttaacta aattgcagaa cctatattta     540

agtaaaaatc acataagcga tttaagagca ttagcaggac ttaaaaatct agatgtttta     600

gaattattta gccaagaatg tcttaataag cctattaatc atcaatctaa tttggttgtt     660

ccgaatacag tgaaaaacac tgatgggtcg ttagtgactc cagaaataat aagtgatgat     720

ggcgattatg aaaaacctaa tgttaaatgg catttaccag aatttacaaa tgaagtgagt     780

tttattttct atcagccagt cactattgga aaagcaaaag caagatttca tgggagagta     840

acccaaccac tgaaa                                                      855

<210>  7
<211>  855
<212>  DNA
<213>  artificial

<220>
<223>  InlB DNA mutated to achieve C242A G206C A227C

<400>  7
gagactatca ccgtgccaac gccaatcaag caaatttttt cagatgatgc ttttgcagaa      60

acaatcaaag acaatttaaa gaaaaaaagt gtgacagatg cagtgacaca aaatgaatta     120

aatagtatag atcaaatcat tgcgaataat agtgatatta aatccgttca aggaattcag     180

tatttaccca atgtgacaaa gttattttta aacgggaata aactaacaga tataaagccc     240

ttagcaaact tgaaaaattt aggatggctt tttttagacg aaaataaagt taaggaccta 300
agttcgctca aggatttaaa aaaattaaaa tcactttctt tggagcataa tggtataagt 360
gatataaacg gacttgttca tttaccacag ctggaaagtt tgtatttggg aaataataaa 420
ataacggata taacggttct ttcacgttta actaaactgg atactttgtc tctcgaagat 480
aaccaaatta gtgatattgt gccacttgca tgtttaacta aattgcagaa cctatattta 540
agtaaaaatc acataagcga tttaagagca ttatgcggac ttaaaaatct agatgtttta 600
gaattattta gccaagaagc tcttaataag cctattaatc atcaatctaa tttggttgtt 660
ccgaatacag tgaaaaacac tgatgggtcg ttagtgactc cagaaataat aagtgatgat 720
ggcgattatg aaaaacctaa tgttaaatgg catttaccag aatttacaaa tgaagtgagt 780
tttatttcct atcagccagt cactattgga aaagcaaaag caagatttca tgggagagta 840
acccaaccac tgaaa 855